# EUROPEAN PATENT APPLICATION

(11) **EP 4 241 776 A1**
(43) Date of publication of application: **13.09.2023**
(21) Application number: 21889608.2
(22) Date of filing: 05.11.2021
(51) Int. Cl.: A61K 35/28, A61P 43/00

(54) **PHARMACEUTICAL COMPOSITION FOR PREVENTING OR TREATING FIBROSIS, COMPRISING ISOLATED MITOCHONDRIA**

(30) Priority: 09.11.2020 KR 20200148232
(71) Applicant: Paean Biotechnology Inc., Daejeon 34013 (KR)
(72) Inventor: KIM, Chun-Hyung, Sejong 30130 (KR); HAN, Kyuboem, Daejeon 34119 (KR); YU, Shin-Hye, Seoul 04736 (KR); KIM, Yu Jin, Seoul 07007 (KR); LEE, Seo-Eun, Suwon-si Gyeonggi-do 16558 (KR); PARK, Jong Hyeok, Seoul 04597 (KR); CHO, Gayoung, Seoul 01180 (KR)
(74) Representative: HGF
(86) International application number: PCT/KR2021/016000
(87) International publication number: WO 2022/098143

(57) **Abstract**

The present invention relates to a pharmaceutical composition for preventing or treating fibrosis and, more specifically, to a pharmaceutical composition for preventing or treating fibrosis, comprising mitochondria as an active ingredient. When the pharmaceutical composition of the present invention comprising exogenous mitochondria as an active ingredient is administered to a subject suffering from fibrosis such as pulmonary fibrosis, hepatic fibrosis and renal fibrosis, the fibrosis of tissues may be inhibited. In addition, the pharmaceutical composition of the present invention effectively inhibits the expression of fibronectin, CTGF2, α-SMA, collagen 1A and the like during treatment of TGF-β. Therefore, the pharmaceutical composition according to the present invention may be effectively used in the prevention or treatment of fibrosis.

## Description

### Technical Field

The present invention relates to a pharmaceutical composition for preventing or treating fibrosis, comprising mitochondria as an active ingredient.

### Background Art

Since fibrosis, which accounts for about 45% of the causes of death in western societies, can be diagnosed after the disease has progressed clinically, there is a limit to preventive treatment. In addition, it is a serious disease field for which there is no effective treatment means to directly address the progressive and pre-formed fibrosis to date. Direct causes of fibrosis include tissue damage, infection, autoimmune disease, organ transplantation and foreign body reaction, tumor, and the like. In addition, aging, obesity, diabetes, cardiovascular disease, hypertension, blood cholesterol, and the like are also pointed out as important causes. Although the causes of fibrosis and the types of diseases expressed are very diverse, it is known that the progression mechanism of fibrosis progressing in each tissue is not only very complex, but also occurs as a result of a complex action of various pathways.

Myofibroblasts, which are abnormally activated fibroblasts, have been pointed out as the causative cells that govern the progression of fibrosis. Myofibroblasts are characterized by accumulation of extracellular matrix (ECM) composed of fibrous type 1,3 collagen and fibronectin in tissues and inactivation of genes encoding enzyme that degrade the extracellular matrix (Dufferield JS, et al., Annu. Rev. Pathol. Mech. Dis, 8:241-276 (2013)).

It is known that growth factors and cytokines, such as TGF-β1 (transforming growth factor-beta 1), TNF-α (tumor necrosis factor-alpha), IL-1 (interleukin-1), IL-6, IL-13, and PDGF (platelet-derived growth factor), which are secreted from damaged epithelium, endothelium, bone marrow-derived fibrin cells, immune cells, and the like, are involved in the differentiation and activation of these myofibroblasts. Therefore, blocking the activation and signaling of the growth factors and cytokines that exacerbate fibrosis can be a starting point for treating fibrosis, and drugs targeting TGF-β1, IL-13, CTGF (connective-tissue growth factor), PDGF, αvβ6 integrin, galectin-3, LOXL2 (lysyl oxidase homolog 2), transglutaminase-2, NOX4 (NADPH oxidase 4) or JNK(Jun N-terminal kinases) inhibitors for the treatment of fibrosis are being developed in the art.

Currently, as drugs administered to patients with idiopathic pulmonary fibrosis, a type of fibrosis, steroids and immunosuppressants are used alone or in combination with steroids and immunosuppressants. However, these drugs show therapeutic efficacy in only about 10 to 30% of all patients, and are not effective with a median survival rate of less than 3 years. In addition, because these drugs have many side effects, the frequency of use is gradually decreasing.

On the other hand, pirfenidone is a drug that inhibits the promoter response of TGF-β. Pirfenidone was a drug that was recommended for conditional use ban in the 2011 guideline, but was recently changed to a drug that can be used conditionally. The drug has been shown to reduce the proliferation of myofibroblasts in animal studies. However, it was reported that in subsequent clinical trials, it has no clear effect, and it has side effects such as nausea, vomiting, and indigestion.

In addition, nintedanib, a tyrosine kinase inhibitor, is a drug that inhibits growth factor receptors of various cells. Nintedanib is known to inhibit the action of cytokines that promote fibrosis at the cellular level, to reduce collagen synthesis by TGF-β, and to alleviate pulmonary fibrosis in fibrosis-induced animal experiments. Although nintedanib has been shown to have the effect of delaying the decline in lung function in a wide range of patients through clinical trials, it is known that it cannot treat pulmonary fibrosis. As such, pirfenidone and nintedanib have limitations in that they do not function as drugs that stop the progress of fibrosis itself.

As such, studies have been conducted to treat fibrosis. However, drugs for treating fibrosis, which are currently being studied, only slow down the progression of the disease, but have a very limited effect and have many side effects. A groundbreaking treatment for fibrosis has not yet been developed. There is a need for continuous research and development on safe and effective therapeutic agents for fibrosis.

On the other hand, mitochondria are cellular organelles of eukaryotic cells involved in the synthesis and regulation of adenosine triphosphate (ATP), an intracellular energy source. Mitochondria are associated with various metabolic pathways *in vivo,* for example, cell signaling, cell differentiation, cell death, as well as control of cell cycle and cell growth. In particular, mitochondria have their own genomes and are organelles that play a central role in the energy metabolism of cells. Mitochondria produce cell energy by the electron transport and oxidative phosphorylation process, and play an important role in being involved in apoptosis signaling pathways.

It has been reported that a reduction in energy production due to a decrease in mitochondrial function induces various human diseases. When the function of the electron transport chain reaction decreases by the variation of the mitochondria genome and proteome, a reduction in ATP production, an excessive production of the reactive oxygen species, a decrease in calcium regulation function in cells and the like occur. In this case, a change in the membrane permeability of the mitochondria occurs, and the function of apoptosis occurs abnormally and may provide the cause of cancer and incurable diseases accordingly. As such, human diseases that result from mitochondrial dysfunction include mitochondria-related genetic disease, heart disease, senile dementia such as Parkinson's disease or Alzheimer's disease, and it has been reported that the development of various cancers and cancer metastasis are also highly related to the abnormal energy metabolism of mitochondria.

### Detailed Description of Invention

### Technical Problem

The present inventors have isolated mitochondria from cells of different species in order to isolate mitochondria exhibiting anti-fibrotic activity, and have compared and analyzed function and anti-fibrotic activity of mitochondria. The present inventors have found that mitochondria isolated from various cells can treat progressive or previously formed fibrosis by inhibiting the activity of TGF-β, which induces the differentiation and proliferation of myofibroblasts. Based on the above, the present inventors completed the present invention.

Therefore, an object of the present invention is to provide a pharmaceutical composition for treating fibrosis and a method for treating fibrosis using the same.

Therefore, another object of the present invention is to provide a pharmaceutical composition for preventing or treating fibrosis, comprising mitochondria exhibiting an anti-fibrotic activity.

### Solution to Problem

In order to solve the above problems, in one aspect of the present invention, there is provided a pharmaceutical composition for preventing or treating fibrosis, comprising mitochondria as an active ingredient.

In another aspect of the present invention, there is provided a method for preventing or treating fibrosis, comprising administering the pharmaceutical composition to a subject.

### Effects of Invention

The pharmaceutical composition according to the present invention comprising mitochondria as an active ingredient inhibits the activity of TGF-β to inhibit the differentiation and proliferation of myofibroblasts, which is the cause of fibrosis, thereby inhibiting the fibrosis of tissues. In addition, the pharmaceutical composition according to the present invention promotes the death of myofibroblasts even after fibrosis has already developed and progressed considerably, thereby promoting the degradation of fibers and their return to normal tissues, and thus may be effectively utilized for the prevention or treatment of fibrosis, an incurable disease.

### Brief Description of Drawings

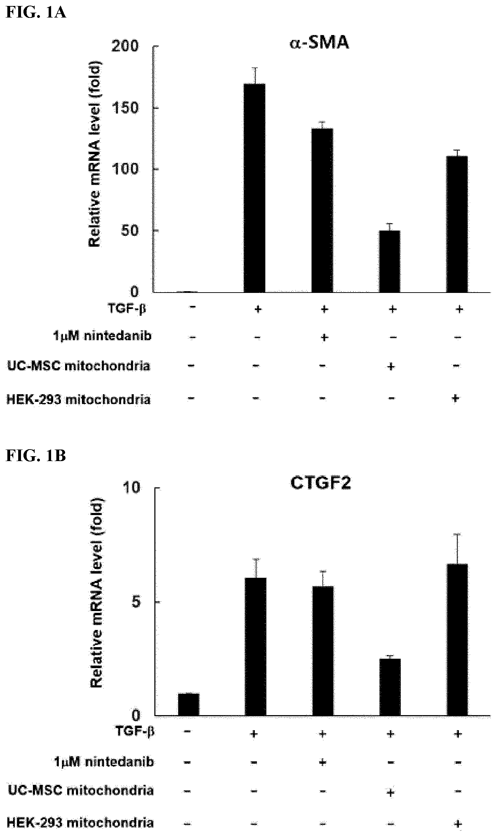

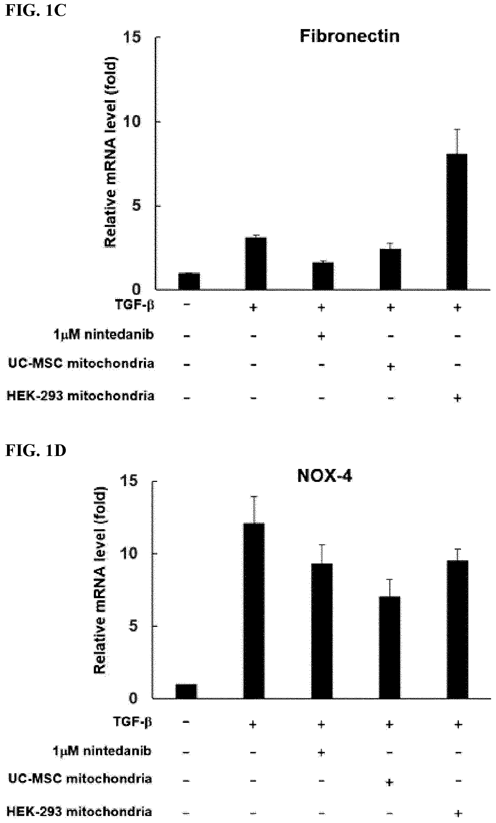

FIG. 1 illustrates the results obtained by comparing the difference in the expression levels of α-SMA gene (FIG. 1A), CTGF gene (FIG. 1B), fibronectin gene (FIG. 1C), and NOX4 gene (FIG. 1D) using quantitative polymerase chain reaction (qRT-PCR) after human lung fibroblast CCD8-Lu cells were treated with TGF-β, nintedanib, mesenchymal stem cell-derived mitochondria and HEK293 cell-derived mitochondria.

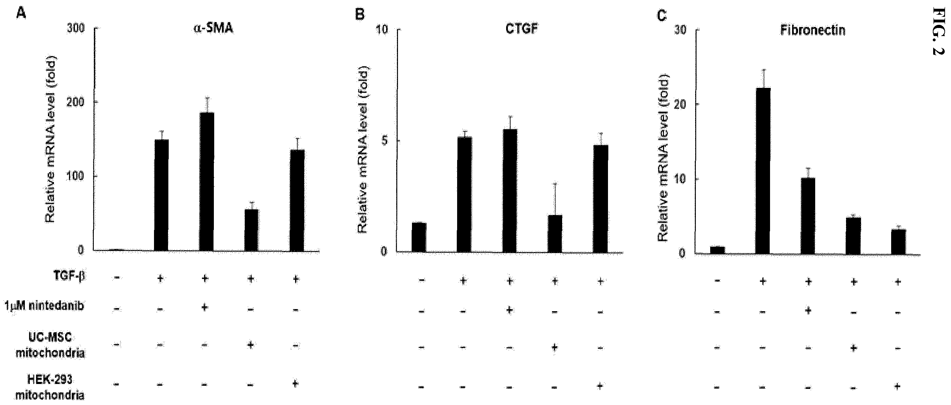

FIG. 2 illustrates the results obtained by comparing the difference in the expression levels of (A) α-SMA gene, (B) CTGF gene, and (C) fibronectin gene using quantitative polymerase chain reaction after human lung fibroblast Wi-38 cells were treated with TGF-β, nintedanib, mesenchymal stem cell-derived mitochondria and HEK293 cell-derived mitochondria.

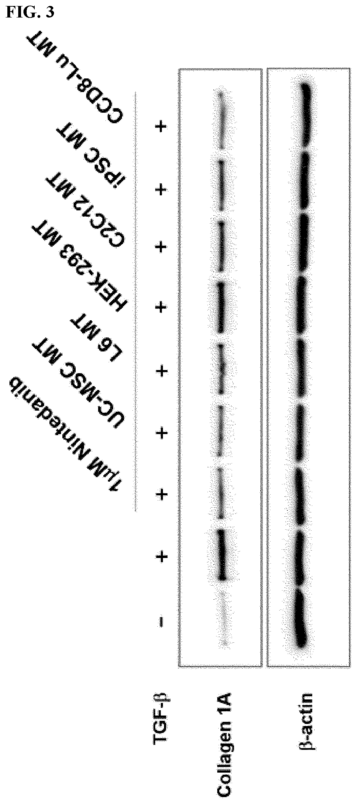

FIG. 3 illustrates the results obtained by comparing the expression level of collagen 1A using western blotting after human lung fibroblast CCD8-Lu cells were treated with TGF-β and/or nintedanib, mesenchymal stem cell-derived mitochondria, L6 cell-derived mitochondria, HEK293 cell-derived mitochondria, C2C12 cell-derived mitochondria, dedifferentiated stem cell-derived mitochondria or human lung fibroblast CCD8-Lu cell-derived mitochondria.

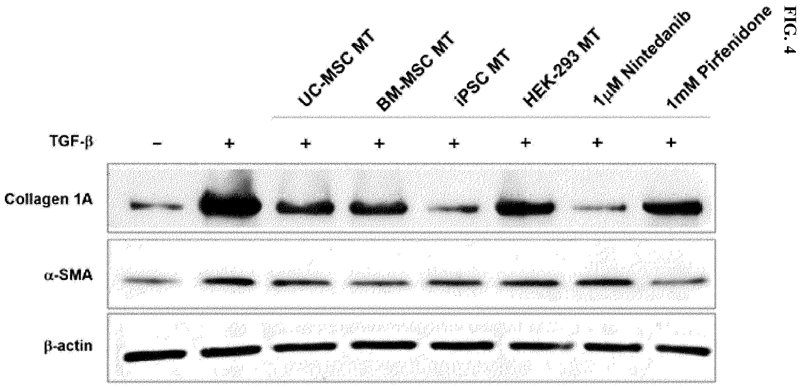

FIG. 4 illustrates the results obtained by comparing the expression levels of collagen 1A and alpha smooth muscle actin protein using western blotting after human lung fibroblast CCD8-Lu cells were treated with TGF-β and/or umbilical cord blood mesenchymal stem cell-derived mitochondria, bone marrow mesenchymal stem cell-derived mitochondria, dedifferentiated stem cell-derived mitochondria, HEK293 cell-derived mitochondria, nintedanib or pirfenidone.

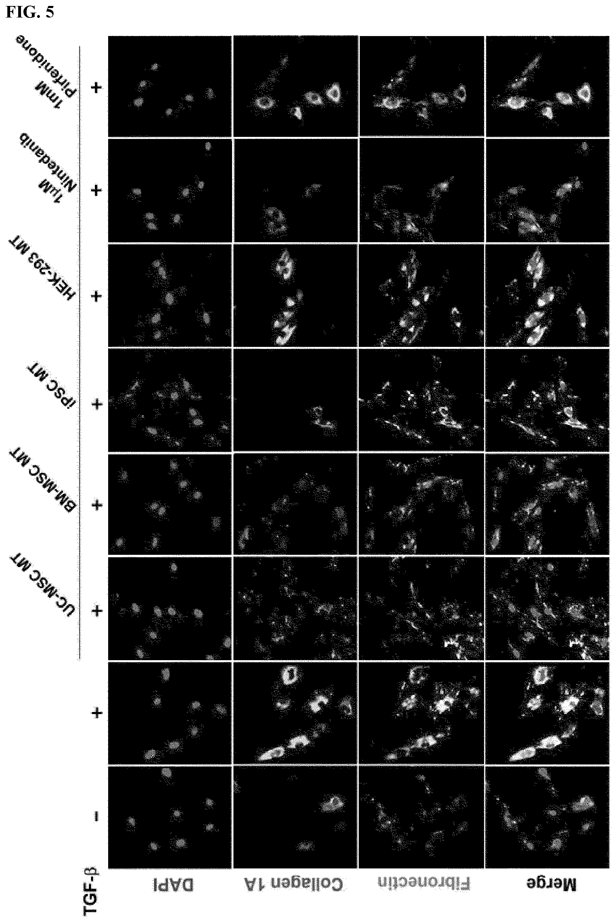

FIG. 5 illustrates the results obtained by comparing the expression levels of collagen 1A and fibronectin protein using an immunocytochemical analysis method after human lung fibroblast CCD8-Lu cells were treated with TGF-β and/or umbilical cord blood mesenchymal stem cell-derived mitochondria, bone marrow mesenchymal stem cell-derived mitochondria, dedifferentiated stem cell-derived mitochondria, HEK293 cell-derived mitochondria, nintedanib or pirfenidone.

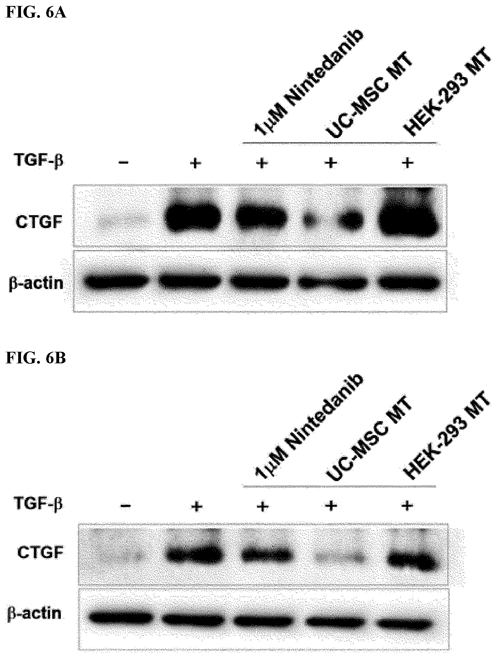

FIG. 6A illustrates the results obtained by comparing the difference in the expression level of CTGF using western blotting after human lung fibroblast CCD8-Lu cells were treated with TGF-β and/or nintedanib, mesenchymal stem cell-derived mitochondria or HEK293 cell-derived mitochondria.
FIG. 6B illustrates the results obtained by comparing the difference in the expression level of CTGF using western blotting after human lung fibroblast Wi-38 cells were treated with TGF-β and/or nintedanib, mesenchymal stem cell-derived mitochondria or HEK293 cell-derived mitochondria.

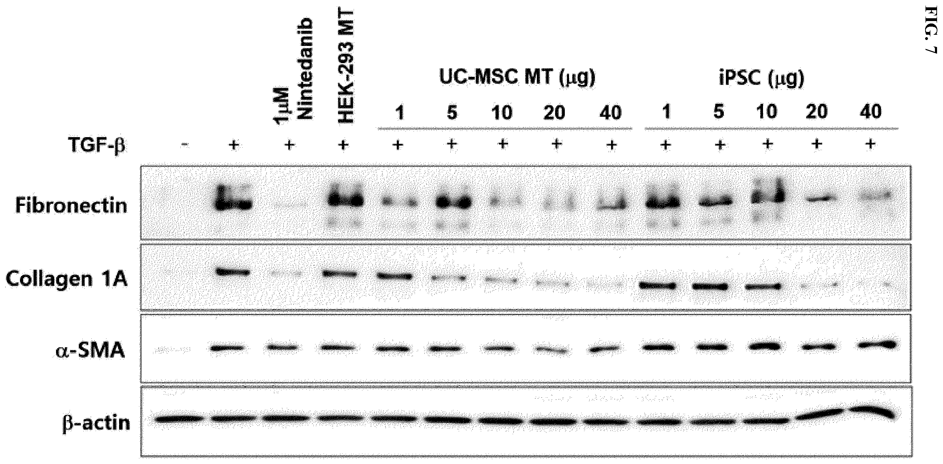

FIG. 7 illustrates the results obtained by comparing the expression levels of fibronectin, collagen 1A and alpha smooth muscle actin using western blotting after human lung fibroblast CCD8-Lu cells were treated with TGF-β and/or nintedanib, HEK293-derived mitochondria and mesenchymal stem cell-derived mitochondria by concentration, dedifferentiated stem cell-derived mitochondria by concentration.

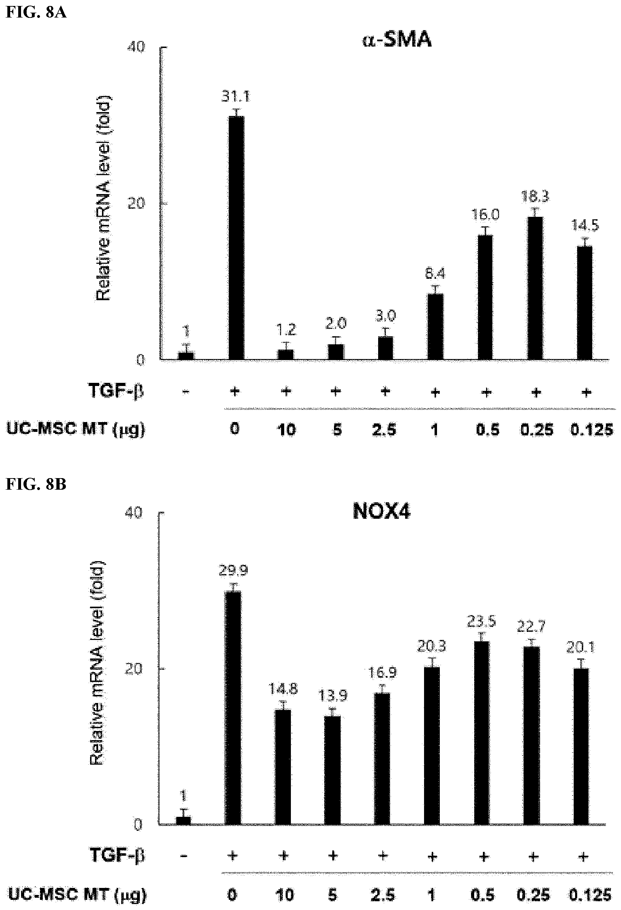

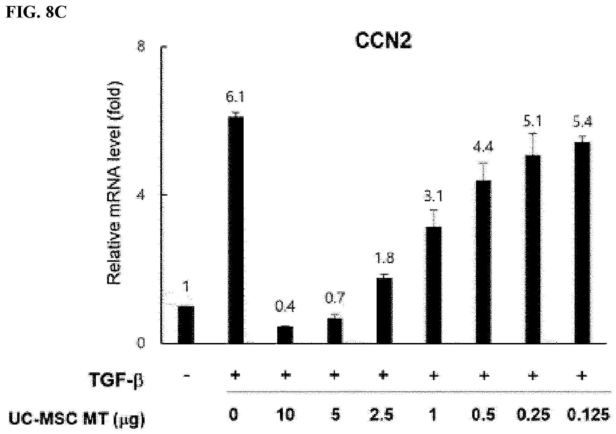

FIGs. 8A, 8B and 8C illustrate the results obtained by comparing the difference in the expression levels of alpha smooth muscle actin, NOX4 and CTGF genes using quantitative polymerase chain reaction after treating with mesenchymal stem cell-derived mitochondria by concentration, respectively.

### Best Mode for Carrying out the Invention

Hereinafter, the present invention will be described in detail.

In one aspect of the present invention, there is provided a pharmaceutical composition for preventing or treating fibrosis, comprising mitochondria as an active ingredient.

As used herein, the term "fibrosis" refers to a disease in which extracellular matrix is abnormally produced, accumulated, and deposited by fibroblasts, and is caused by fibrosis of organs or tissues. Fibrosis refers to a very fatal disease that induces organ damage. In addition, it is used interchangeably with "fibrotic condition", "fibroproliferative condition", "fibrotic disease", "fibroproliferative disease", "fibrotic disorder" and "fibroproliferative disorder", and it can refer to a condition, disease or disorder characterized by dysregulated proliferation or activity of fibroblasts, abnormal accumulation of fibronectin and/or pathological or excessive accumulation of collagenous tissue.

The fibrosis may occur in any one or more selected from the group consisting of kidney, liver, lung, skin, heart, pancreas, urinary system, reproductive system, sweat gland, nerve, brain, bone marrow, muscle and joint. Specifically, the fibrosis may be any one selected from the group consisting of pulmonary fibrosis, idiopathic pulmonary fibrosis, radiation-induced lung injury or pulmonary fibrosis, pulmonary edema, cystic fibrosis, hepatic fibrosis, endomyocardial fibrosis, myocardial infarction, artrial fibrosis, glial scar, renal fibrosis, myelofibrosis, arthrofibrosis, fat fibrosis, skin fibrosis, nerve fibrosis and muscle fibrosis.

As used herein, the term "pulmonary fibrosis" refers to the development of scarred (fibrous) tissue due to the formation or development of excessive fibrous connective tissue (fibrosis) in the lungs. Specifically, pulmonary fibrosis is a chronic disease that causes swelling and scarring of the alveoli and interstitial tissue of the lungs. Such scarred tissue replaces healthy tissue and causes inflammation, and chronic inflammation may be identified as a precursor to fibrosis. Due to such damage to the lung tissue, the lungs may become stiff, and it may become difficult for the subject to breathe on their own.

In the present specification, pulmonary fibrosis may be pulmonary fibrosis caused by idiopathic pulmonary fibrosis, radiation-induced lung injury, nonspecific interstitial pneumonia, acute interstitial pneumonia, cryptogenic organizing pneumonia, respiratory bronchiolitis associated interstitial lung disease, desquamative interstitial pneumonia, lymphoid interstitial pneumonia, interstitial pulmonary fibrosis, and diffuse pulmonary fibrosis, pulmonary edema, cystic fibrosis or metabolic disease.

As used herein, the term "renal fibrosis" includes all diseases in which fibrosis occurs in the kidney due to various causes, and fibrosis may be renal fibrosis caused by catheter installation, glomerulosclerosis, glomerulonephritis, nephritis, acute renal failure, chronic renal failure, end-stage renal disease or metabolic disease.

As used herein, the term "hepatic fibrosis (liver fibrosis)" refers to a symptom in which fibrous tissue proliferates due to chronic liver damage. Specifically, the hepatic fibrosis may be caused by any one selected from the group consisting of chronic liver disease, hepatitis B virus infection, hepatitis C virus infection, hepatitis D virus infection, schistosomiasis, alcoholic liver disease or non-alcoholic steatohepatitis, metabolic disease, protein deficiency, coronary artery disease, autoimmune hepatitis, cystic fibrosis, alpha-1 antitrypsin deficiency, primary biliary cirrhosis, drug reaction and toxins.

Unless otherwise specified in the present specification, "active ingredient" refers to a component that exhibits activity alone or in combination with an auxiliary agent (carrier) that is inactive by itself.

As used herein, the term "mitochondria" are cellular organelles of eukaryotic cells involved in the synthesis and regulation of adenosine triphosphate (ATP), an intracellular energy source. Mitochondria are involved in various metabolic pathways *in vivo.*

The mitochondria may be obtained from eukaryotes, and may be obtained from mammals or humans. Specifically, the mitochondria may be isolated from cells or tissues. For example, the mitochondria may be isolated from cells cultured *in vitro.* In addition, the mitochondria may be obtained from somatic cells, germ cells, blood cells, or stem cells. In addition, the mitochondria may be obtained from platelets. The mitochondria may be normal mitochondria obtained from cells having normal mitochondrial biological activity. In addition, the mitochondria may be cultured *in vitro.* In addition, the mitochondria may be isolated after disruption by concentrating the tissues or cells, or may be isolated after disruption from a tissue or cell sample thawed after freezing and storage.

In addition, the mitochondria may be obtained from an autologous, allogenic, or xenogenic subject. Specifically, the autologous mitochondria refer to mitochondria obtained from tissues or cells of the same subject. In addition, the allogenic mitochondria refer to mitochondria obtained from a subject that belongs to the same species as the subject and has different genotypes for alleles. In addition, the xenogenic mitochondria refer to mitochondria obtained from a subject that belongs to the different species from the subject.

Specifically, the somatic cells may be muscle cells, hepatocytes, nerve cells, fibroblasts, epithelial cells, adipocytes, osteocytes, leukocytes, lymphocytes, platelets, or mucosal cells. In addition, the germ cell is a cell that undergoes meiosis and mitosis and may be a sperm or an egg. In addition, the stem cells may be any one selected from the group consisting of mesenchymal stem cells, adult stem cells, dedifferentiated stem cells, embryonic stem cells, bone marrow stem cells, neural stem cells, limbal stem cells, and tissue-derived stem cells. In this case, the mesenchymal stem cells may be any one selected from the group consisting of umbilical cord, umbilical cord blood, bone marrow, fat, muscle, nerve, skin, amniotic membrane and placenta. Preferably, the mitochondria may be obtained from mesenchymal stem cells derived from umbilical cord blood.

On the other hand, when the mitochondria are isolated from specific cells, the mitochondria may be isolated by a variety of modified methods, including various known methods, for example, using a specific buffer solution or using a potential difference and a magnetic field and the like.

The mitochondrial isolation may be obtained by disrupting cells and centrifuging in terms of maintaining mitochondrial activity.

The isolated mitochondria may be quantified by quantifying proteins of the mitochondria. Specifically, the isolated mitochondria may be quantified by BCA (Bicinchoninic acid Assay) analysis method. In this case, the mitochondria may be included at a concentration of 0.1 µg/mL to 1,000 µg/mL, 1 µg/mL to 750 µg/mL, or 25 µg/mL to 500 µg/mL in the pharmaceutical composition. In one embodiment of the present invention, it was used at a concentration of 25 µg/mL, 50 µg/mL, and 100 µg/mL.

In addition, the number of the isolated mitochondria may be measured by a particle counter (Multisizer 4e, Beckman Coulter). Referring to the paper written by James D. McCully (J Vis Exp. 2014; (91): 51682.), the number of mitochondria may be as shown in Table 1 below.

**[Table 1]**

| Amount of isolated mitochondria (µg) | Number of mitochondria | Concentration (µg/mL) |
|---|---|---|
| 0.01 | 2.16×10⁵ ± 0.01×10⁵ | 0.1 |
| 1 | 2.16×10⁷ ± 0.08×10⁷ | 10 |
| 25 | 0.54×10⁹ ± 0.02×10⁹ | 250 |
| 50 | 1.08×10⁹ ± 0.04×10⁹ | 500 |
| 100 | 2.16×10⁹ ± 0.08×10⁹ | 1,000 |

In the pharmaceutical composition of the present invention for preventing or treating fibrosis, the active ingredient may be included in any amount (effective amount) depending on the purpose of use, formulation, combination, and the like, as long as it may exhibit a preventive or therapeutic effect on fibrosis. Here, "effective amount" refers to the amount of an active ingredient capable of inducing a preventive or therapeutic effect on fibrosis. Such an effective amount may be determined experimentally within the ordinary skill of those of ordinary skill in the art.

The mitochondria in the pharmaceutical composition may be included in an amount of 1×10⁵ mitochondria/mL to 5×10⁹ mitochondria/mL. Specifically, the mitochondria in the pharmaceutical composition may be included in an amount of 1×10⁵ mitochondria/mL to 5×10⁹ mitochondria/mL, 2X10⁵ mitochondria/mL to 2×10⁹ mitochondria/mL, 5X10⁵ mitochondria/mL to 1×10⁹ mitochondria/mL, 1×10⁶ mitochondria/mL to 5X10⁸ mitochondria/mL, 2×10⁶ mitochondria/mL to 2×10⁸ mitochondria/mL, 5X10⁶ mitochondria/mL to 1×10⁸ mitochondria/mL or 1×10⁷ mitochondria/mL to 5X10⁷ mitochondria/mL. The pharmaceutical composition comprises mitochondria at a concentration and amount within the above range, thereby facilitating mitochondria dose regulation upon administration and further enhancing the degree of improvement of fibrosis in a patient.

As used herein, the term "prevention" refers to any action of inhibiting the development or delaying the onset of fibrosis by administration of the pharmaceutical composition. In addition, "treatment" refers to any action in which fibrosis is improved or beneficially changed by administration of the pharmaceutical composition.

The pharmaceutical composition of the present invention is administered to a subject in a pharmaceutically effective amount. The term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined depending on factors including the health condition of the patient, the type and severity of the disease, the activity of the drug, sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective to treat fibrosis. The preferred dosage of the pharmaceutical composition may vary depending on the condition and body weight of the patient, the severity of disease, the form of drug, the route and duration of administration, but may be appropriately selected by those of ordinary skill in the art.

As used herein, the term "administration" refers to introducing a predetermined substance to a subject by an appropriate method, and the route of administration of the composition may be through any general route as long as it may reach a target tissue. It may be intraperitoneal administration, intravenous administration, intramuscular administration, subcutaneous administration, intradermal administration, topical administration, intranasal administration, intrarectal administration. In addition, it may be administered intranasally via inhalation.

The preferred dosage of the mitochondria included in the pharmaceutical composition may be 3×10⁵ mitochondria/kg to 1.5×10¹⁰ mitochondria/kg per dose based on the body weight of the subject to be administered. Specifically, the therapeutically effective dose of the mitochondria in the pharmaceutical composition may be 3×10⁵ mitochondria/kg to 1.5×10¹⁰ mitochondria/kg, 6×10⁵ mitochondria/kg to 6×10⁹ mitochondria/kg, 1.5X10⁶ mitochondria/kg to 3×10⁹ mitochondria/kg, 3×10⁶ mitochondria/kg to 1.5X10⁹ mitochondria/kg, 6×10⁶ mitochondria/kg to 6×10⁸ mitochondria/kg, 1.5X10⁷ mitochondria/kg to 3×10⁸ mitochondria/kg or 3×10⁷ mitochondria/kg to 1.5X10⁸ mitochondria/kg per dose based on the body weight of the subject to be administered. That is, it is most preferable in terms of cell activity that the pharmaceutical composition comprising the mitochondria is administered with the mitochondria in a dose in the above range based on the body weight of the subject having fibrosis.

In addition, the pharmaceutical composition may be administered 1 to 10 times, 3 to 8 times, or 5 to 6 times, preferably 5 times. In this case, the administration interval may be an interval of 1 to 7 days or 2 to 5 days, preferably an interval of 3 days.

The term "subject" refers to a subject to which the pharmaceutical composition of the present invention may be applied (prescribed), and may be a subject that may develop fibrosis or suffer from fibrosis. In addition, the subject may be a mammal, such as a rat, a mouse, or a livestock, including a human, and preferably a human. The composition of the present invention may further comprise any compound or natural extract known to have fibrosis improving effect and safety, which has already been verified, for the enhancement and reinforcement of fibrosis improving effect, in addition to the mitochondria. In this case, the pharmaceutical composition and the compound or natural extract having fibrosis improving effect may be administered simultaneously or sequentially.

The pharmaceutical composition of the present invention may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmaceutically acceptable adjuvant (buffering agent, dispersing agent) may be included in the pharmaceutical composition.

Specifically, the pharmaceutical composition may be prepared as a parenteral formulation depending on the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the application (prescription) target may adapt.

When the pharmaceutical composition of the present invention is prepared as a parenteral formulation, it may be formulated in the form of an injection, a transdermal preparation and a nasal inhalant together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used.

The pharmaceutical composition of the present invention may be an injectable preparation that may be administered intravenously, intramuscularly, subcutaneously or mucosally. Therefore, the pharmaceutical composition according to the present invention may be manufactured as an injection that is very stable physically or chemically by adjusting the pH using a buffer solution such as an acidic aqueous solution or phosphate, which may be used as an injection, in order to secure product stability according to the distribution of an injection that is prescribed.

Specifically, the pharmaceutical composition of the present invention may comprise water for injection. The water for injection refers to distilled water prepared for dissolving a solid injection or for diluting a water-soluble injection.

In addition, the pharmaceutical composition of the present invention may comprise a stabilizer or a solubilizing agent. For example, the stabilizer may be pyrosulfite, citric acid or ethylenediamine tetraacetic acid, and the solubilizing agent may be hydrochloric acid, acetic acid, sodium hydroxide, sodium bicarbonate, sodium carbonate or potassium hydroxide.

In one embodiment of the pharmaceutical composition of the present invention, it may be a liquid composition for injection comprising glycine, sugar, buffer and mitochondria. In this case, the sugar included in the liquid composition for injection of the present invention may be at least one selected from the group consisting of sucrose, trehalose, mannitol, sorbitol, glucose, fructose, mannose, maltose, lactose, isomaltose, dextran and dextrin, but are not limited thereto. In particular, the sugar may be trehalose, mannitol or sucrose. Preferably, the sugar may be trehalose. In this case, the buffer included in the liquid composition for injection of the present invention may be selected from the group consisting of a Tris buffer, a HEPES (Hydroxyethyl piperazine Ethane Sulfonic acid) buffer, a MOPS (3-(N-morpholino)propane sulfonic acid) buffer and an acetate- or phosphate-containing buffer, but is not limited thereto. Preferably, the buffer may be a Tris buffer.

In another aspect of the present invention, the present invention provides a method for preventing or treating fibrosis, comprising administering the aforementioned pharmaceutical composition to a subject. Here, the subject may be a mammal that is likely to suffer from fibrosis or suffers from fibrosis, and the subject may be preferably a human. Details on mitochondria, fibrosis, prevention and treatment are as described above.

In this case, the administration may be intravenous, intramuscular, or intradermal administration. Given this, the pharmaceutical composition according to the present invention may supply exogenous mitochondria having normal activity to the veins of subjects suffering from fibrosis, and thus is useful for increasing the activity of cells with reduced mitochondrial function or for regenerating cells with abnormal mitochondrial function, and may be used for the prevention or treatment of fibrosis.

In another aspect of the present invention, there is provided a use of isolated mitochondria for the prevention or treatment of fibrosis. Details on mitochondria, fibrosis, prevention and treatment are as described above.

### Mode for Carrying out the Invention

Hereinafter, preferred embodiments will be presented to help the understanding of the present invention. However, the following examples are provided only to more easily understand the present invention, and the content of the present invention is not limited to the following examples.

### Preparation Example 1. Cell culture and mitochondria isolation

HEK293, L6 and C2C12 cells were cultured in DMEM medium containing 10% FBS. In addition, umbilical cord blood-derived mesenchymal stem cells, UC-MSC, and bone marrow-derived mesenchymal stem cells, BM-MSC cells, were cultured in alpha MEM medium containing 10% FBS and 20 ng/mL bFGF. Dedifferentiated stem cells, iPSC cells, were cultured in TeSR-E8 medium using a plate coated with 10 µg/mL vitronectin.

After the culture was completed, the cells were washed twice using DPBS (Dulbecco's Phosphate Buffered Saline, Gibco). The washed cells were treated with 0.25% (v/v) Trypsin-EDTA (TE, Gibco) to obtain the cells. In order to isolate mitochondria from the obtained cells, the cells were collected at a concentration of 1×10⁷ cells/mL using a hemocytometer. The cell line was subjected to first centrifugation at a speed of 350 x g at 4°C for 10 minutes. At this time, the obtained pellet was collected, resuspended in a buffer solution, and then homogenized for 10 to 15 minutes. Thereafter, the composition comprising the pellet was subjected to second centrifugation at a speed of 1,100 x g at 4°C for 3 minutes to obtain a supernatant. The supernatant was subjected to third centrifugation at a speed of 12,000 x g at 4°C for 15 minutes to isolate mitochondria from the cell line. The isolated mitochondria were suspended in a buffer and used for experiments after protein quantification by the BCA method.

### Experimental Example 1. Analysis of anti-fibrotic activity of mitochondria

### Experimental Example 1.1. Comparison of anti-fibrotic activity using quantitative real-time polymerase chain reaction

In order to analyze the anti-fibrotic activity of mitochondria, a cell-based analysis experiment was performed using a quantitative real-time polymerase chain reaction.

CCD8-Lu cells and Wi-38 cells, which are human lung-derived fibroblasts, were cultured in DMEM medium containing 10% FBS, respectively. The cells were inoculated into a 6 well plate at about 2X10⁵ cells/well and cultured for 24 hours, and then depletion conditions were performed in DMEM medium without FBS for about 24 hours. After 24 hours, the cells were treated with TGF-β at a concentration of 2.5 ng/mL, and then fibrosis of the cells was induced for 6 hours. After 6 hours, the cells were washed twice with PBS to completely remove the remaining TGF-β. Next, the mitochondria isolated from HEK293, UC-MSC and BM-MSC cells in Preparation Example 1 were mixed in DMEM medium, and the cells were treated with the mitochondria and then further cultured for 18 hours (FIG. 1). At this time, the negative control group was not treated with anything, the positive control group was treated with TGF-β alone at a concentration of 5 ng/mL, and the experimental groups were treated with 10 µg of mitochondria isolated from each cell, respectively. Anti-fibrotic efficacy by mitochondria was compared by treatment with nintedanib, which is used as a conventional anti-fibrotic therapeutic agent, at a concentration of 1 µM.

After 18 hours of mitochondria treatment, the culture solution was removed, and the cells were washed twice with a PBS buffer solution, and 0.5 mL of RNA extract (Trizol reagent, Thermo Fisher Scientific) was added directly and then stood at ambient temperature for 10 minutes. Thereafter, 0.1 mL of chloroform was added and stirred for 15 seconds, and then centrifuged at about 12,000 x g for 10 minutes. The separated supernatant was obtained, and the same volume of isopropyl alcohol was added and then centrifuged at 12,000 x g for 10 minutes. Thereafter, the liquid was removed, washed once with 75% ethanol, and then dried at ambient temperature. After drying, about 50 µl of purified RNAase-free distilled water was added, and the quantity and purity of the obtained RNA was measured using a spectrophotometer.

In order to synthesize cDNA using the obtained RNA, 2 µg of purified total RNA was subjected to a binding reaction with oligo dT at 70°C for 5 minutes, and then 10X reverse transcription reaction buffer solution, 10 mM dNTP, RNAse inhibitor and M-MLV reverse transcriptase (Enzynomics, Korea) were added, and cDNA synthesis reaction was performed at 42°C for 60 minutes. After the cDNA synthesis reaction was completed, reverse transcriptase was inactivated by heating at 72°C for 5 minutes, and then single-stranded RNA was removed by adding RNase H to obtain final cDNA.

Changes in the expression of the alpha actin smooth muscle (α-SMA) gene, a characteristic gene of myofibroblast, the CCN2 (or CTGF) gene, a characteristic gene of fibrosis, fibronectin, a characteristic gene of fibrosis, and the NOX-4 gene, a characteristic gene of fibrosis, were observed by quantitative real-time polymerase chain reaction. The GAPDH gene was quantified together to calibrate for differences in the expression. The nucleotide sequences of the genes used in the quantitative real-time polymerase chain reaction are as shown in the table below.

**[Table 2]**

| Primer | Sequence | SEQ ID NO |
|---|---|---|
| CTGF-S | 5'-GGC TTA CCG ACT GGA AGA C-3' | SEQ ID NO: 1 |
| CTGF-AS | 5'-AGG AGG CGT TGT CAT TGG-3' | SEQ ID NO: 2 |
| Fibronectin-S | 5'-GTG GCT GAA GAC ACA AGG AA-3' | SEQ ID NO: 3 |
| Fibronectin-AS | 5'-CCT GCC ATT GTA GGT GAA T-3' | SEQ ID NO: 4 |
| NOX4-S | 5'- CAC CTC TGC CTG TTC ATC TG-3' | SEQ ID NO: 5 |
| NOX4-AS | 5'- GGC TCT GCT TAG ACA CAA TCC-3' | SEQ ID NO: 6 |
| Alpha-SMA-S | 5'-GCC CAG CCA AGC ACT GTC AGG A-3' | SEQ ID NO: 7 |
| Alpha-SMA-AS | 5'-TCC CAC CAT CAC CCC CTG ATG TC-3' | SEQ ID NO: 8 |
| GAPDH-S | 5'-GTC TCC TCT GAC TTC AAC AGC G-3' | SEQ ID NO: 9 |
| GAPDH-AS | 5'-ACC ACC CTG TTG CTG TAG CCA A-3' | SEQ ID NO: 10 |

As a result of the experiment, as shown in FIGs. 1 and 2, it was found that when CCD8-Lu cells (FIGs. 1A to 1D) and Wi-38 cells (FIG. 2), which are human lung-derived fibroblasts, were treated with TGF-β, the expression of alpha actin smooth muscle (α-SMA), CTGF, fibronectin and NOX-4 genes was increased. In addition, it was found that the expression of alpha actin smooth muscle (α-SMA), CTGF, fibronectin and NOX-4 genes induced by TGF-β was significantly inhibited when treated with mesenchymal stem cell-derived mitochondria.

On the other hand, it was found that HEK293 cell-derived mitochondria did not inhibit the expression of alpha actin smooth muscle (α-SMA), CTGF, fibronectin and NOX-4 genes induced by TGF-β. It was found that the nintedanib compound used as an anti-fibrotic therapeutic agent weakly inhibited or hardly inhibited the expression of alpha actin smooth muscle (α-SMA), CTGF and NOX-4 genes induced by TGF-β. Given this, it was found that mesenchymal stem cell-derived mitochondria exhibited significantly excellent anti-fibrotic activity under the same conditions compared to that of the nintedanib compound, which is known as a conventional therapeutic agent for pulmonary fibrosis. In addition, it was found that it exhibited excellent anti-fibrotic activity compared to that of the HEK293 cell-derived mitochondria.

### Experimental Example 1.2. Comparison of anti-fibrotic activity by expression of fibrosis-related protein

### Experimental Example 1.2.1. Comparison of anti-fibrotic activity by mitochondria derived from various cells

In order to analyze the anti-fibrotic activity of mitochondria, a cell-based analysis experiment was performed using a western analysis method.

CCD8-Lu cells, which are human lung-derived fibroblasts, were cultured in DMEM medium containing 10% FBS. The cells were inoculated into a 6 well plate at about 2X10⁵ cells/well and cultured for 24 hours, and then depletion conditions were performed in DMEM medium without FBS for about 24 hours. After 24 hours, the cells were treated with TGF-β at a concentration of 2.5 ng/mL, and then fibrosis of the cells was induced for 6 hours. After 6 hours, the cells were washed twice with PBS to completely remove TGF-β. Next, the mitochondria isolated from UC-MSC, L6, HEK293, C2C12, iPSC and CCD8-Lu cells obtained in Preparation Example 1 were mixed in DMEM medium, and the cells were treated with the mitochondria and then further cultured for 18 hours (FIG. 3). At this time, the negative control group was not treated with anything, the positive control group was treated with TGF-β alone at a concentration of 2.5 ng/mL, and the experimental groups were treated with 5 µg of mitochondria isolated from each cell, respectively.

After 18 hours of mitochondria treatment, the culture solution was removed, and the cells were washed twice with a PBS buffer solution, and 100 µl of RIPA (150 mM NaCl, 1% NP-40, 0.5% deoxycholic acid, 0.1% SDS, and 50 mM Tris pH 7.5) buffer solution containing a protease inhibitor was added directly. After 10 minutes, the cells were collected using a scraper, transferred to a microtube, and then centrifuged at about 12,000 x g for 10 minutes.

The separated supernatant was obtained and transferred to a new microtube, and then protein was quantified using the BCA analysis method. The same amount of protein for each sample was electrophoresed, and then western blotting was performed. An anti-collagen 1A antibody (Invitrogen, PA5-29569), an anti-fibronectin antibody (Santa Cruz Biotechnology, sc-8422), an anti-smooth muscle actin antibody (Sigma, A5228), or an anti-CTGF antibody (Santa Cruz Biotechnology, sc-365970) was used as a primary antibody, and an anti-mouse IgG HRP antibody or an anti-rabbit IgG HRP antibody was used as a secondary antibody. For calibration, differences in protein expression were calibrated using an anti-β actin antibody (Sigma, A2228) antibody.

As a result of the experiment, as shown in FIG. 3, it was found that when human lung-derived fibroblasts, CCD8-Lu, were treated with TGF-β, the expression of collagen 1A was increased, and it was found that when treated with mitochondria derived from various cells including mesenchymal stem cell-derived mitochondria, the expression of collagen 1A induced by TGF-β was inhibited. On the other hand, it was found that when treated with HEK293 cell-derived mitochondria, the expression of the collagen 1A protein induced by TGF-β was hardly inhibited. Given this, it was found that the mitochondria derived from various cells including mesenchymal stem cells and dedifferentiated stem cells exhibited an anti-fibrotic activity at a level similar to that of nintedanib, which is known as a conventional therapeutic agent for pulmonary fibrosis.

### Experimental Example 1.2.1. Comparison of anti-fibrotic activity by stem cell-derived mitochondria

In order to analyze the anti-fibrotic activity of stem cell-derived mitochondria, a cell-based analysis experiment was performed using a western analysis method.

CCD8-Lu cells, which are human lung-derived fibroblasts, were cultured in DMEM medium containing 10% FBS. The cells were inoculated into a 6 well plate at about 2X10⁵ cells/well and cultured for 24 hours, and then depletion conditions were performed in DMEM medium without FBS for about 24 hours. After 24 hours, the cells were treated with TGF-β at a concentration of 2.5 ng/mL, and then fibrosis of the cells was induced for 6 hours. After 6 hours, the cells were washed twice with PBS to completely remove TGF-β. Next, the mitochondria isolated from umbilical cord blood mesenchymal stem cell UC-MSC, bone marrow mesenchymal stem cell BM-MSC, dedifferentiated stem cell iPSC and HEK293 cells obtained in Preparation Example 1 were mixed in DMEM medium, and the cells were treated with the mitochondria and then further cultured for 18 hours (FIG. 4). At this time, the negative control group was not treated with anything, the positive control group was treated with TGF-β alone at a concentration of 2.5 ng/mL, and the experimental groups were treated with 5 µg of mitochondria isolated from each cell, respectively.

After 18 hours of mitochondria treatment, the culture solution was removed, and the cells were washed twice with a PBS buffer solution, and 100 µl of RIPA (150 mM NaCl, 1% NP-40, 0.5% deoxycholic acid, 0.1% SDS, and 50 mM Tris pH 7.5) buffer solution containing a protease inhibitor was added directly. After 10 minutes, the cells were collected using a scraper, transferred to a microtube, and then centrifuged at about 12,000 x g for 10 minutes. The separated supernatant was obtained and transferred to a new microtube, and then protein was quantified using the BCA analysis method. The same amount of protein for each sample was electrophoresed, and then western blotting was performed. An anti-collagen 1A antibody or an anti-alpha smooth muscle actin antibody was used as a primary antibody, and an anti-mouse IgG HRP antibody or an anti-rabbit IgG HRP antibody was used as a secondary antibody. For calibration, differences in protein expression were calibrated using an anti-β actin antibody.

As a result of the experiment, as shown in FIG. 4, it was found that when human lung-derived fibroblasts, CCD8-Lu, were treated with TGF-β, the expression of collagen 1A and alpha actin smooth muscle (α-SMA) was increased, and it was found that the expression of collagen 1A and alpha actin smooth muscle (α-SMA) induced by TGF-β was inhibited by the mitochondria derived from umbilical cord blood mesenchymal stem cells, bone marrow mesenchymal stem cells and dedifferentiated stem cells. On the other hand, it was found that when treated with HEK293 cell-derived mitochondria, the expression of collagen 1A and alpha actin smooth muscle (α-SMA) induced by TGF-β was hardly inhibited.

Given this, it was found that the mitochondria derived from stem cells including mesenchymal stem cells and dedifferentiated stem cells exhibited an anti-fibrotic activity at a level superior to or similar to that of nintedanib and pirfenidone, which are known as conventional therapeutic agents for pulmonary fibrosis.

### Experimental Example 1.3. Analysis of anti-fibrotic activity using cell immunochemical staining analysis method

In order to analyze the anti-fibrotic activity of stem cell-derived mitochondria, a cell-based analysis experiment was performed using a western analysis method.

CCD8-Lu cells, which are human lung-derived fibroblasts, were cultured in DMEM medium containing 10% FBS. Thereafter, the cells were inoculated into a 24 well plate at about 2×10⁴ cells/well, cultured for 24 hours, and then reacted in DMEM medium without FBS for about 24 hours. After 24 hours, the cells were treated with TGF-β at a concentration of 2.5 ng/mL, and then fibrosis of the cells was induced for 6 hours. After 6 hours, the cells were washed twice with PBS to completely remove TGF-β. Next, the mitochondria isolated from umbilical cord blood mesenchymal stem cell UC-MSC, bone marrow mesenchymal stem cell BM-MSC, dedifferentiated stem cell iPSC and HEK293 cells obtained in Preparation Example 1 were mixed in DMEM medium, and the cells were treated with the mitochondria and then further cultured for 18 hours (FIG. 4). At this time, the negative control group was not treated with anything, the positive control group was treated with TGF-β alone at a concentration of 2.5 ng/mL, and the experimental groups were treated with 5 µg of mitochondria isolated from each cell, respectively.

Immunohistochemical analysis for each sample was performed as follows. First, after washing twice with PBS buffer, 4% formaldehyde was added and fixed for 1 hour, and an anti-collagen 1A antibody and an anti-fibronectin antibody were used as a primary antibody, and an anti-mouse antibody or an anti-rabbit antibody labeled with Alexa Fluor 488 or Alexa Fluor 568 was used as a secondary antibody. The nucleus was stained using DAPI (4,6-diamidino-2-phenylindole), and analysis of differences in fluorescence intensity was performed using a fluorescence microscope.

As a result of the experiment, as shown in FIG. 4, it was found that when human lung-derived fibroblasts, CCD8-Lu, were treated with TGF-β, the expression of collagen 1A and alpha actin smooth muscle (α-SMA) was increased, and it was found that the expression of collagen 1A and alpha actin smooth muscle (α-SMA) induced by TGF-β was inhibited by treatment with the mitochondria derived from umbilical cord blood mesenchymal stem cells, bone marrow mesenchymal stem cells and dedifferentiated stem cells. On the other hand, when treated with HEK293 cell-derived mitochondria, the expression of collagen 1A and alpha actin smooth muscle (α-SMA) induced by TGF-β was hardly inhibited. Given this, it was found that the mitochondria derived from stem cells including mesenchymal stem cells and dedifferentiated stem cells exhibited similar or significantly superior anti-fibrotic activity compared to that of nintedanib and pirfenidone, which are known as conventional therapeutic agents for pulmonary fibrosis.

As a result of the experiment, as shown in FIG. 5, it was found that by the intensity of fluorescence, the expression of collagen 1A and fibronectin was low in CCD8-Lu cells, the negative control group, and the expression of collagen 1A and fibronectin was significantly increased when treated with 2.5 ng/mL TGF-β. In addition, it was found that after 6 hours of treatment with 2.5 ng/mL of TGF-β, when treated with the mitochondria derived from umbilical cord blood mesenchymal stem cells, bone marrow mesenchymal stem cells and dedifferentiated stem cells, the increase in the expression of collagen 1A and fibronectin induced by TGF-β was significantly inhibited when treated with the mitochondria derived from umbilical cord blood mesenchymal stem cells, bone marrow mesenchymal stem cells and dedifferentiated stem cells.

On the other hand, it was found that when treated with the HEK293 cell-derived mitochondria and 1 mM of the pirfenidone compound, the expression of the collagen 1A and fibronectin proteins induced by TGF-β was weakly inhibited or hardly inhibited. Given this, it was found that stem cell-derived mitochondria exhibited similar or significantly excellent anti-fibrotic activity under the same conditions compared to that of the nintedanib compound and the pirfenidone compound, which are known as conventional therapeutic agents for pulmonary fibrosis.

### Experimental Example 1.4. Comparison of anti-fibrotic activity by inhibition of CTGF expression

In order to observe whether the expression of CTGF, a key gene for fibrosis, is regulated by mesenchymal stem cell-derived mitochondria, a cell-based analysis experiment was performed using a Western analysis method.

CCD8-Lu cells (FIG. 6A) and Wi-38 cells (FIG. 6B), which are human lung-derived fibroblasts, were cultured in DMEM medium containing 10% FBS. The cells were inoculated into a 6 well plate at about 2X10⁵ cells/well and cultured for 24 hours, and then depletion conditions were performed in DMEM medium without FBS for about 24 hours.

After 24 hours, the cells were treated with TGF-β at a concentration of 2.5 ng/mL, and then fibrosis of the cells was induced for 6 hours. After 6 hours, the cells were washed twice with PBS to completely remove TGF-β. Next, in the same manner as in Preparation Example 1, the mitochondria isolated from UC-MSC and HEK293 cells were mixed in DMEM medium, and the cells were treated with the mitochondria and then further cultured for 18 hours (FIG. 3). At this time, the negative control group was not treated with anything, the positive control group was treated with TGF-β alone at a concentration of 2.5 ng/mL, and the experimental groups were treated with 5 µg of mitochondria isolated from each cell, respectively.

After 18 hours of mitochondria treatment, the culture solution was removed, and the cells were washed twice with a PBS buffer solution, and 100 µl of RIPA (150 mM NaCl, 1% NP-40, 0.5% deoxycholic acid, 0.1% SDS, 50 mM Tris pH 7.5) buffer solution containing a protease inhibitor was added directly. After 10 minutes, the cells were collected using a scraper, transferred to a microtube, and then centrifuged at about 12,000 x g for 10 minutes.

The separated supernatant was obtained and transferred to a new microtube, and then protein was quantified using the BCA analysis method. The same amount of protein for each sample was electrophoresed, and then western blotting was performed. An anti-CTGF antibody was used as a primary antibody, and an anti-rabbit IgG HRP antibody was used as a secondary antibody. For calibration, differences in protein expression were calibrated using an anti-β actin antibody.

As a result of the experiment, as shown in FIG. 6, it was found that when human lung-derived fibroblasts, CCD8-Lu, were treated with TGF-β, the expression of CTGF was increased, and it was found that when treated with mesenchymal stem cell-derived mitochondria, the expression of CTGF induced by TGF-β was inhibited. On the other hand, it was found that when treated with HEK293 cell-derived mitochondria and nintedanib, an anti-fibrotic therapeutic agent, the expression of the CTGF protein induced by TGF-β was hardly inhibited. Given this, it was found that mesenchymal stem cell-derived mitochondria exhibited an anti-fibrotic activity at an excellent level compared to that of nintedanib, which is known as a conventional therapeutic agent for pulmonary fibrosis.

### Experimental Example 1.5. Comparison of anti-fibrotic activity depending on concentration of stem cell-derived mitochondria

In order to analyze whether the anti-fibrotic activity of the mitochondria derived from mesenchymal stem cells and dedifferentiated stem cells changes depending on the amount of mitochondria to be used for treatment, a cell-based analysis experiment was performed using a western analysis method.

CCD8-Lu cells, which are human lung-derived fibroblasts, were cultured in DMEM medium containing 10% FBS. The cells were inoculated into a 6 well plate at about 2X10⁵ cells/well and cultured for 24 hours, and then depletion conditions were performed in DMEM medium without FBS for about 24 hours. After 24 hours, the cells were treated with TGF-β at a concentration of 2.5 ng/mL, and then fibrosis of the cells was induced for 6 hours. After 6 hours, the cells were washed twice with PBS to completely remove TGF-β. Next, 1 µg, 5 µg, 10 µg, 20 µg or 50 µg of the mitochondria isolated from umbilical cord blood mesenchymal stem cell UC-MSC and dedifferentiated stem cell iPSC obtained in Preparation Example 1 were mixed in DMEM medium, respectively, and the cells were treated with the mitochondria and then further cultured for 18 hours (FIG. 7). At this time, the negative control group was not treated with anything, the positive control group was treated with TGF-β alone at a concentration of 2.5 ng/mL. The comparison group was treated with 1 µM nintedanib and the mitochondria derived from HEK293 cells.

After 18 hours of mitochondria treatment, the culture solution was removed, and the cells were washed twice with a PBS buffer solution, and 100 µl of RIPA (150 mM NaCl, 1% NP-40, 0.5% deoxycholic acid, 0.1% SDS, 50 mM Tris pH 7.5) buffer solution containing a protease inhibitor was added directly. After 10 minutes, the cells were collected using a scraper, transferred to a microtube, and then centrifuged at about 12,000 x g for 10 minutes.

The separated supernatant was obtained and transferred to a new microtube, and then protein was quantified using the BCA analysis method. The same amount of protein for each sample was electrophoresed, and then western blotting was performed. An anti-fibronectin antibody, an anti-collagen 1A antibody or an anti-alpha smooth muscle actin antibody was used as a primary antibody, and an anti-mouse IgG HRP antibody or an anti-rabbit IgG HRP antibody was used as a secondary antibody. For calibration, differences in protein expression were calibrated using an anti-β actin antibody.

As a result of the experiment, as shown in FIG. 7, it was found that when human lung-derived fibroblasts, CCD8-Lu, were treated with TGF-β, the expression of fibronectin, collagen 1A and alpha actin smooth muscle (α-SMA) was increased, and it was found that the expression of fibronectin, collagen 1A and alpha actin smooth muscle (α-SMA) induced by TGF-β was inhibited in a concentration dependent manner by the mitochondria derived from umbilical cord blood mesenchymal stem cells and dedifferentiated stem cells. Given this, it was found that the mitochondria derived from stem cells including mesenchymal stem cells and dedifferentiated stem cells exhibited an anti-fibrotic activity in a concentration dependent manner.

## Claims

1. A pharmaceutical composition for preventing or treating fibrosis, comprising mitochondria as an active ingredient.

2. The pharmaceutical composition for preventing or treating fibrosis according to claim 1, wherein the fibrosis occurs in any one or more selected from the group consisting of kidney, liver, lung, skin, heart, pancreas, urinary system, reproductive system, sweat gland, nerve, brain, bone marrow, muscle and joint.

3. The pharmaceutical composition for preventing or treating fibrosis according to claim 1, wherein the fibrosis is any one selected from the group consisting of pulmonary fibrosis, idiopathic pulmonary fibrosis, radiation-induced lung injury or pulmonary fibrosis, pulmonary edema, cystic fibrosis, hepatic fibrosis, endomyocardial fibrosis, myocardial infarction, artrial fibrosis, glial scar, renal fibrosis, myelofibrosis, arthrofibrosis, fat fibrosis, skin fibrosis, nerve fibrosis and muscle fibrosis.

4. The pharmaceutical composition for preventing or treating fibrosis according to claim 1, wherein the mitochondria are isolated from cells or tissues.

5. The pharmaceutical composition for preventing or treating fibrosis according to claim 1, wherein the mitochondria are isolated from any one selected from the group consisting of mesenchymal stem cells, adult stem cells, dedifferentiated stem cells, embryonic stem cells, bone marrow stem cells, neural stem cells, limbal stem cells, tissue-derived stem cells, platelets and a combination thereof.

6. The pharmaceutical composition for preventing or treating fibrosis according to claim 1, wherein with respect to the pharmaceutical composition, the mitochondria are included at a concentration of 0.1 µg/mL to 1,000 µg/mL.

7. The pharmaceutical composition for preventing or treating fibrosis according to claim 1, wherein the mitochondria are included in an amount of 1×10⁵ to 5X10⁸ mitochondria/mL with respect to the pharmaceutical composition.

8. A method for preventing or treating fibrosis, comprising administering the pharmaceutical composition according to any one of claims 1 to 7 to a subject.

9. Use of isolated mitochondria for the prevention or treatment of fibrosis.
